# EUROPEAN PATENT APPLICATION

(11) **EP 1 563 836 A1**
(43) Date of publication of application: **17.08.2005**
(21) Application number: 03755696.6
(22) Date of filing: 08.10.2003
(51) Int. Cl.: A61K 31/351, A61K 9/06, A61K 9/10, A61P 17/00, A61P 35/00, A61P 43/00, C07D 309/32

(54) **DRUG FOR INHIBITING PRODUCTION OF MATRIX METALLOPROTEASE-9**

(30) Priority: 20.11.2002 JP 2002336867; 15.05.2003 JP 2003136738; 05.08.2003 JP 2003286386
(71) Applicant: Japan Science and Technology Agency, Kawaguchi-shi, Saitama 332-0012 (JP)
(72) Inventor: KOBAYASHI, Takashi, 901 Chibachuou Sunny Court, Chiba-shi, Chiba 260-0021 (JP)
(74) Representative: Hakvoort, Ansgar
(86) International application number: PCT/JP2003/012898
(87) International publication number: WO 2004/045602

(57) **Abstract**

A drug for inhibiting the production of matrix metalloproteinase-9 which contains leptomycin B or its derivative as the active ingredient can specifically inhibit the production of MMP-9 compared with MMP-2, furthermore, has an effect of inhibiting the production of MMP-9 even under stimulating the differentiation by adding calcium at a high concentration or adding TGF-β, and under stimulating for inducing inflammation by adding TNF-α or adding IL-1α.

## Description

### Technological Field

The present invention relates to a drug for inhibiting the production of matrix metalloproteinase-9.

### Background Technology

Leptomycin B (or LMB) of the following formula (1) 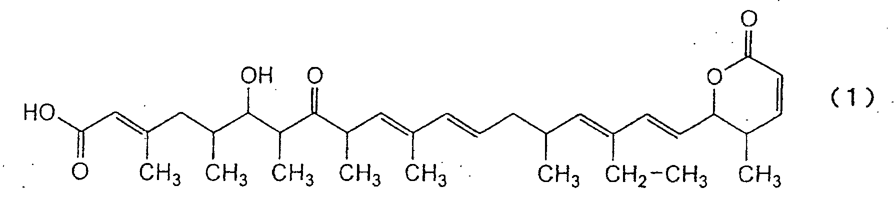 has been found originally as an anti-fungal antibiotic, however, thereafter, it has been clarified that also a derivative of the leptomycin B has analogous anti-fungal activity, and currently, it is paid to attention particularly as an anti-cancer agent.

Japanese Patent Application Laid-Open (JP-A) No. 5-13133 describes an anti-tumor agent containing leptomycinB as an active ingredient and administered by parenteral administration methods such as subcutaneous injection, intravenous injection, intramuscular injection and the like or oral administration methods. This anti-tumor agent can show a strong anti-tumor action on P388 cell, Lewis Landa carcinoma cell, B16 melanoma cell, Erich carcinoma cell and the like transplanted to mouse, however, since it is not clear how leptomycin B acts on a tumor cell, applications other than anti-tumor agents have not been developed.

Matrix metalloproteinases (MMPs) are a generic name for a group of metalloproteinases having an extracellular matrix protein as a main substrate. MMPs play an important role in tissue metabolism in vivo. Among them, matrix metalloproteinase-9 (MMP-9) not only acts as gelatinase to decompose gelatin components but also decomposes extracellular matrix components such as various collagens, elastin, fibronectin and the like as called alias type IV collagenase, and further, a possibility of activating a transforming growth factor (TGF-β) and a tumor necrosis factor (TNF-α) has been shown.

Recently, from investigation on mouse in which MMP-9 has been artificially deleted and investigation on mouse in which expression of MMP-9 has been monitored, it has been clarified that particularly MMP-9 infiltrated from peripheral inflammatory cells is a factor causing infiltration and metastasis of cancer and at the same time a factor causing blister formation in bullosis, and that MMP-9 derived from epidermal is correlated with apoptosis in irradiation with ultraviolet ray and consequently with ultraviolet dermatitis. It has also been clarified that MMP-9 causes delay of injury healing. Further, it has been clarified that MMP-9 causes neovascularization. In addition, since the above-described bullosis, ultraviolet dermatitis and delay of injury healing are skin inflammatory diseases, MMP-9 correlated with these skin inflammatory diseases is believed to relate to eczematous dermatitis.

Regarding a relation between MMP-9 derived from epidermal and epidermal keratinization, the present inventors have separated and purified MMP-9 derived from neutrophiles, produced a monoclonal antibodies against MMP-9, and investigated localization of MMP-9 in skin tissue by this antibody, and resultantly confirmed localization of MMP-9 in epidermal keratinocyte tissue. Further, it has been confirmed that expression of MMP-9 is promoted more specifically as compared with MMP-2 in stimulating differentiation into keratinocytes using cultured epidermal keratinocyte. Though it has been found that KRE-M9 which is a gene promoter region common to involucrin which is a marker for keratinization is present in an MMP-9 promoter, in addition to conventionally known TPA responsive element, as a transcription factor binding gene region regarding this promotion of expression, this finding supports promotion of specific expression of MMP-9 in stimulating differentiation into keratinocyte. Originally, keratinization is programmed cell death of an epidermal keratinocyte and believed to be one of apoptosis, and in such apoptosis, particularly, in apoptosis of epidermal cells, clinical conditions of hyperkeratosis, namely, dyskeratosis are often manifested as one inflammation condition, and since the study of the present inventors has clarified that expression of MMP-9 is specifically promoted in stimulating differentiation into keratinocyte, a possibility has been suggested of correlation of MMP-9 also in dyskeratosis in such apoptosis in epidermal keratinization.

Thus, also in the dermatology region, MMP-9 is correlated with skin inflammatory diseases such as infiltration and metastasis of cancer, blister formation in bullosis, neovascularization, delay of injury healing, eczematous dermatitis, ultraviolet dermatitis, dyskeratosis and the like.

Consequently, improvement of skin inflammatory diseases such as infiltration and metastasis of cancer, bullosis, neovascularization, delay of injury healing, eczematous dermatitis, ultraviolet dermatitis, dyskeratosis and the like by inhibiting the production of MMP-9 is expected.

Therefore, an object of the present invention is to provide a novel drug inhibiting the production of MMP-9.

### DISCLOSURE OF THE INVENTION

Under such conditions, the present inventors have intensively studied and resultantly found that the leptomycin B can inhibit the production of MMP-9 derived from cultured epidermal keratinocyte more specifically as compared with MMP-2, further that the leptomycin B can inhibit the production of MMP-9 even under stimulating the differentiation by adding calcium at a high concentration, or adding TGF-β, and under stimulating for inducing inflammation by adding TNF-α or adding IL-1α, leading to completion of the invention.

Namely, the present invention (1) provides a drug for inhibiting the production of matrix metalloproteinase-9, comprising leptomycin B of the following formula (1) 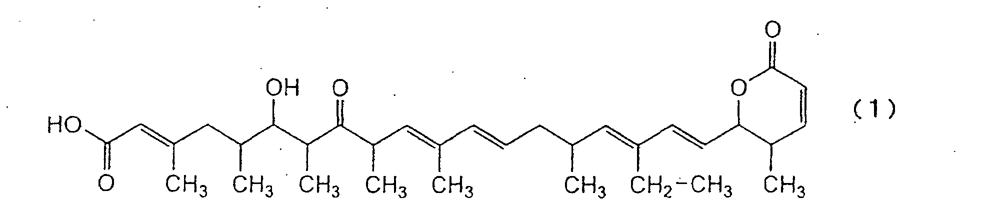 or its derivative as the active ingredient. Such a constitution performs an effect that the production of MMP-9 can be inhibited specifically as compared with MMP-2, further, the production of MMP-9 can be inhibited even under stimulating the differentiation by adding calcium at a high concentration or adding TGF-β, and under stimulating for inducing inflammation by adding TNF-α or adding IL-1α.

Further, the present invention (2) provides the drug according to the above-mentioned invention (1), which is a preventive drug or a therapeutic drug for matrix metalloproteinase-9 related skin diseases. This constitution performs an effect that prevention or treatment of metalloproteinase-9 related skin diseases can be conducted effectively, in addition to the effect performed by the above-mentioned invention (1).

Furthermore, the present invention (3) provides the drug according to the above-mentioned invention (2) wherein the above-mentioned preventive drug or therapeutic drug is administered in the form of skin external medicine. This constitution performs an effect that an effect of preventing or treating MMP-9 related skin diseases can be further improved, in addition to the effect performed by the above-mentioned invention (2).

### BRIEF EXPLANATION OF DRAWINGS

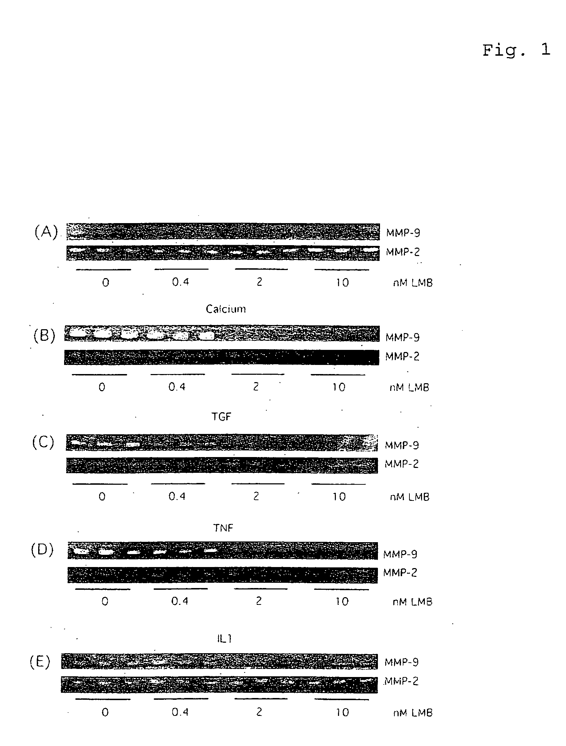

Fig. 1 provides views showing an influence of LMB exerted on the production of MMP-2 and MMP-9 of a human epidermal keratinocyte, (A) under no stimulation, (B) under stimulation by calcium of high concentration, (C) under stimulation by TGF-β, (D) under stimulation by TNF-α, and (E) under stimulation by IL-1α.

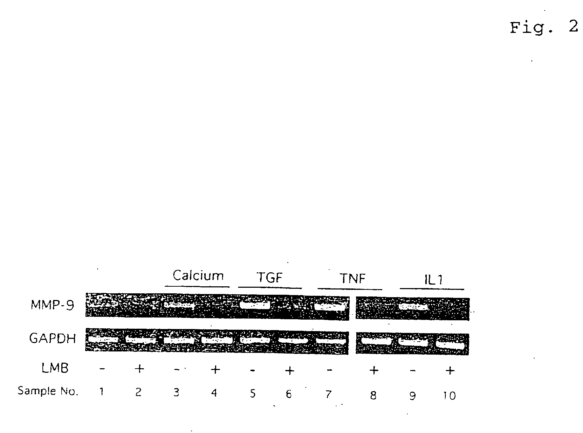

Fig. 2 is a view showing an influence of LMB exerted on the production of mRNA of MMP-9 and mRNA of GAPDH of a human epidermal keratinocyte.

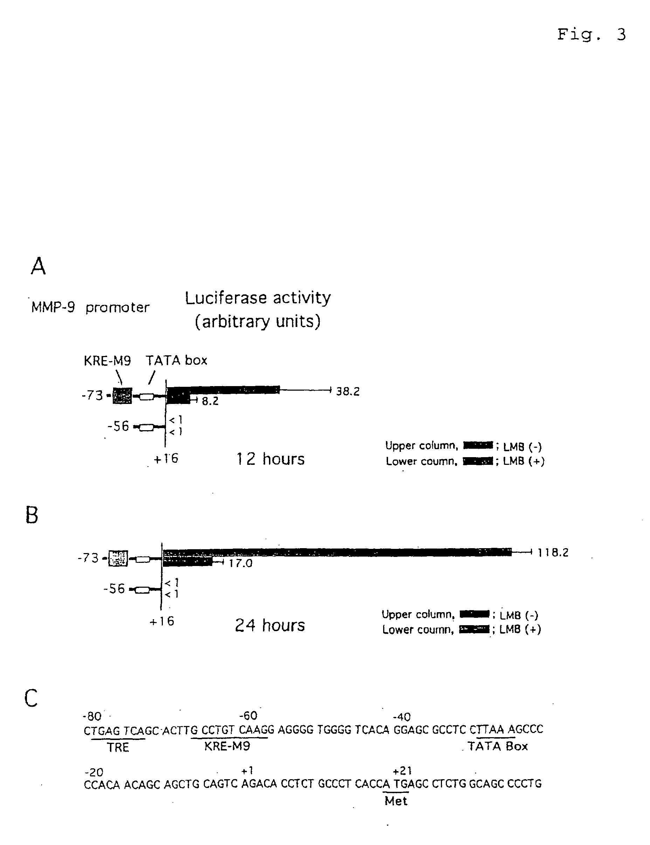

Fig. 3 (A) and (B) are views showing the results of measurement of total transcription activity of MMP-9 of a human epidermal keratinocyte by luciferase assay in the case of gene introduction of vector 1 containing KRE-M9 into HFKs and in the case of gene introduction of vector 2 containing no KRE-M9 into HFKs, and Fig. 3 (C) is a view showing a base sequence containing a KRE-M9 sequence and a TRE sequence.

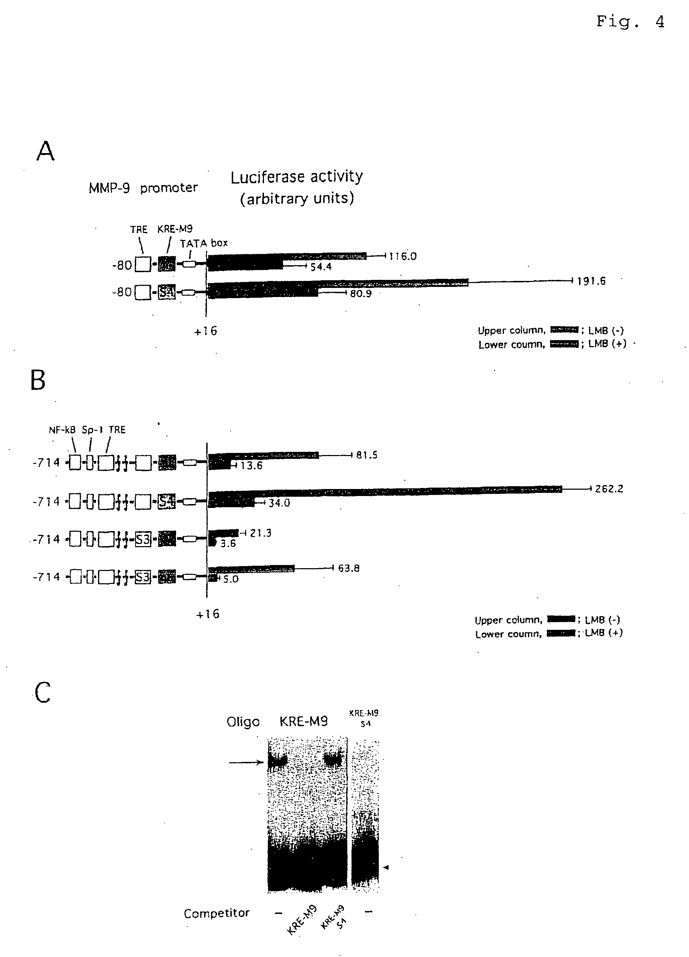

Fig. 4 (A) and (B) are views showing the results of measurement of total transcription activity of MMP-9 of a human epidermal keratinocyte by luciferase assay in the case of use of base sequences 1 and 2 and in the case of use of base sequences 3 to 6, and Fig. 4 (C) is a view showing the results of gel shift assay by a human keratinocyte nucleus protein using as a probe a KRE-M9 sequence and a 4 base-substituted sequence of a KRE-M9 sequence as depicted in SEQ ID No. 5.

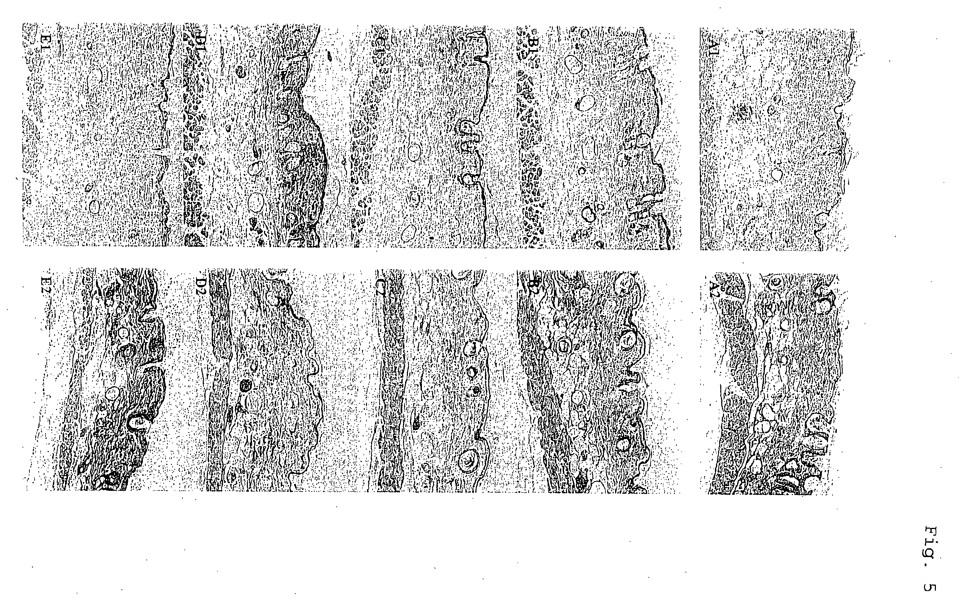

Fig. 5 is a view showing the results of observation by an optical microscope of slices of skin cut from mice A1 to E1 and stained, and slices of skin cut from mice A2 to E2 and stained.

### BEST MODES FOR CARRYING OUT THE INVENTION

The drug for inhibiting MMP-9 of the present invention contains LMB or its derivative as the active ingredient. LMB is an antibiotic produced from an LMB production germ belonging to Streptomyces belonging to Actinomycetes and can be obtained by separation and purification from a cultured LMB production germ according to a method described in (The Journal of Antibiotics, 1983, vol. 36, pp. 639 to 650).

As the LMB derivative, there exemplified derivatives of LMB selected from the group consisting of leptomycin A of the following formula (2) 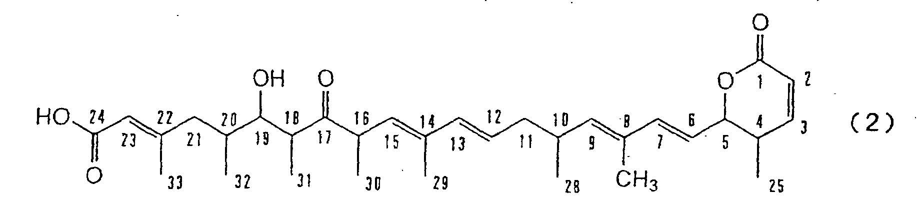 a substance S-59917a of the following formula (3) 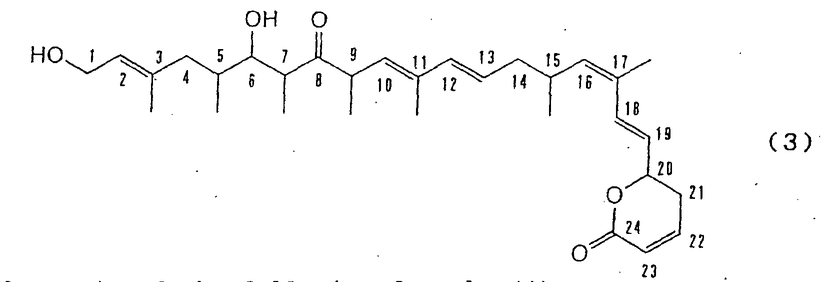 and elactocin of the following formula (4)

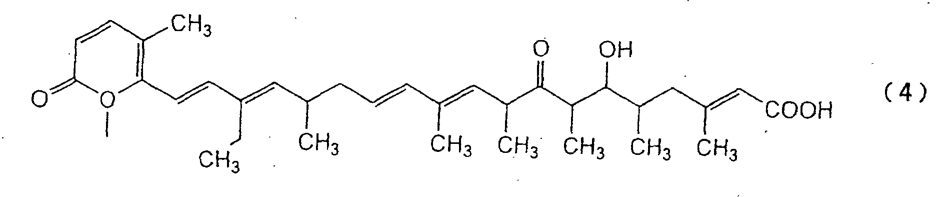

Leptomycin A is an antibiotic produced from the above-mentioned LMB production germ and can be obtained by separation and purification from the above-mentioned cultured LMB production germ according to a method described in (The Journal of Antibiotics, 1983, vol. 36, pp. 639 to 650). The substance S-59917a is an antibiotic produced from a substance S-59917a production germ belonging to Streptomyces, and can be obtained by separation and purification from a cultured S-59917a production germ as described in Japanese Patent Application Laid-Open (JP-A) No. 5-39283.

The drug for inhibiting the production of MMP-9 of the present invention can be used as a preventive drug or a therapeutic drug for MMP-9 related skin diseases, and the MMP-9 related skin diseases include infiltration and metastasis of cancer, bullosis, neovascularization, delay of injury healing, eczematous dermatitis, ultraviolet dermatitis, dyskeratosis and the like, preferably, bullosis, neovascularization, delay of injury healing, eczematous dermatitis, ultraviolet dermatitis, or dyskeratosis, particularly preferably bullosis, delay of injury healing, eczematous dermatitis, ultraviolet dermatitis, or dyskeratosis. The drug for inhibiting the production of MMP-9 of the present invention suppresses the production of MMP-9, and it is also preferable that, in the case of induction of the production of MMP-9 by causing an inflammatory reaction in a cell by cytokines such as TNF-α, IL-1α and the like, the drug inhibits the production of MMP-9. In this case, the present drug is effective as a therapeutic drug or a preventive drug for MMP-9 related skin diseases such as bullosis, neovascularization, delay of injury healing, eczematous dermatitis and the like, or a therapeutic drug or a preventive drug for MMP-9 related skin diseases such as bullosis, delay of injury healing, eczematous dermatitis and the like. Further, also in the case of induction of the production of MMP-9 by inducing a differentiation such as keratinization and the like in a cell by differentiation stimulation by adding calcium at a high concentration or TGF-β, it is preferable that the production of MMP-9 is inhibited, and in this case, the present drug is effective as a therapeutic drug or a preventive drug for MMP-9 related skin diseases such as ultraviolet dermatitis, dyskeratosis and the like.

When the drug of the present invention is used as a medicine, it may be advantageous that leptomycin B or its derivative in an amount effective for preventive or therapeutic object is formulated together with a pharmaceutically acceptable carrier or diluent. In addition, a binder, absorption promoter, lubricant, emulsifer, surfactant, antioxidant, preservative, coloring agent, aromatic, sweetener or the like may be added. The dosage form of the medical preparation includes granule, fineparticle, tablet, capsule, pill, ointment, gel, paste, cream, spray, solution, suspension and the like, and the dosage embodiments thereof include oral administration, and additionally, various administration embodiments such as parenteral administrations such as injection, external application and the like.

The preparation of the present invention is preferably a therapeutic drug or a preventive drug which is administered in the form of external preparation, and particularly preferably a skin external preparation which is applied on skin. For the drug of the present invention to be used in the form of skin external preparation, LMB or its derivative, and in addition, components compounded in external preparations for usual drug or cosmetics, for example, ultraviolet absorbers, oily components, humectants, thickening agents, emulsifiers, preservatives, powders, emulsification stabilizers, pH regulators, aromatics, alcohols, water and the like can be compounded. Here, the ultraviolet absorber includes organic ultraviolet absorbers such as dibenzoylmethane derivatives, cinnamate ester derivatives, benzophenone derivatives, p-aminobenzoic acid derivatives and the like, and inorganic ultraviolet absorbers such as zinc oxide, mica, mica titanium, titanium oxide, magnesium oxide, zioconium oxide and the like.

The oily components include liquid paraffin, Vaseline, paraffin wax, squalane, beeswax, carnauba wax, oliveoil, lanolin, higher alcohols, fatty acids, synthetic ester oils of higher alcohols with fatty acids, silicone oils, fluorine-based oils and the like.

The compounding amount of leptomycin B or its derivative into a skin external preparation is not particularly restricted, and preferably from 0.0001 to 50 wt%, particularly preferably from 0.001 to 20 wt%. The skin external preparation of the present invention may be any drug used as a skin external preparation such as an ointment, cream, emulsion, lotion, pack, bath preparation and the like, and the dosage form is not particularly restricted.

### EXAMPLES

The present invention will be illustrated further specifically by the following examples, but these are only exemplary and do not limit the invention.

### Example 1

The following experiment was conducted to verify that the production of MMP-9 is inhibited by LMB.

### <Culturing of human epidermal keratinocyte>

A foreskin of a neonate was treated at 4°C for 16 hours by a disperse having a casein decomposition activity of 25.0 caseinolytic units/ml. Epidermal was peeled from dermis using a forceps, then, the epidermal was treated with 0.05% trypsin for 5 minutes. The separated human foreskin keratinocytes (HFKs) were allowed to incubate at 37°C in a plate having a keratinocyte-SFM medium (manufactured by Invitrogen), and sub-cultured over three generations. Then, into four plates obtained by sub-culturing human foreskin keratinocytes (HFKs) over three generations, LMB was added so that the concentrations in the medium were 0 nM, 0.4 nM, 2 nM and 10 nM, respectively, further, allowed to incubate at 37°C for 24 hours. Then, four conditioned media of the keratinocyte-SFM medium were collected from the fourplates, and stored at -30°C until use in the following detection of MMP-2 and MMP-9 by a gelatin zymography method.

### <Detection of MMP-2 and MMP-9 by gelatin zymography method>

Prior to detection of MMP-2 and MMP-9 by a gelatin zymography method, four conditioned media collected in <Culturing of human epidermal keratinocyte cell> were all allowed to incubate at 35°C for 1 hour. Then, the above-mentioned conditioned media were dissolved in a solution of pH 7.4 containing 0 . 05 M Tris-HCl, 5 mM CaCl₂, 1% SDS (sodium dodecyl-sulfate) and 5% glycerol, respectively, and the solutions were used as samples for a gelatin zymography method. MMP-2 and MMP-9 were separated from the samples by an SDS-PAGE method using 7.5% polyacrylamide gel containing 0.5% gelatin. After separation by an SDS-PAGE method, the separated gel was washed with a solution containing 2.5% Triton X-100 for 1 hour, to remove SDS. For allowing MMP-2 and MMP-9 separated in the separated gel to decompose gelatin in the separated gel, the separated gel was allowed to incubate at 35°C for a given time in abuffer solution for reaction (standard conditions: 0.05 M Tris-HCl (pH 7.4)/0.15 M NaCl/5 mM CaCl₂/0.02% NaN₃). Staining of the separated gel was conducted using a staining solution containing 0.1% Amideblack B-10, and decolorization was conducted using a decolorization solution containing 10 vol% of acetic acid and 30 vol% of methanol. The results of detection of MMP-2 and MMP-9 in the four conditioned media are shown in Fig. 1 (A).

### Example 2

Culturing of a human epidermal keratinocyte and detection of MMP-2 and MMP-9 by a gelatin zymography method were conducted by the same manner as in Example 1 except that LMB was added into four plates obtained by sub-culturing human foreskin keratinocytes (HFKs) over three generations, so that the concentrations in the medium were 0 nM, 0.4 nM, 2 nM and 10 nM, respectively, and a calcium chloride aqueous solution was added so that the four concentrations of Ca⁺ in the medium were all 1.5 mM, instead of adding LMB into four plates obtained by sub-culturing human foreskin keratinocytes (HFKs) over three generations, so that the concentrations in the medium were 0 nM, 0.4 nM, 2 nM and 10 nM, respectively, in culturing of human epidermal keratinocyte. The results are shown in Fig. 1 (B).

### Example 3

Culturing of a human epidermal keratinocyte and detection of MMP-2 and MMP-9 by a gelatin zymography method were conducted by the same manner as in Example 1 except that LMB was added into four plates obtained by sub-culturing human foreskin keratinocytes (HFKs) over three generations, so that the concentrations in the medium were 0 nM, 0.4 nM, 2 nM and 10 nM, respectively, and TGF-β was added so that the four concentrations of TGF-β in the medium were all 1 ng/ml, instead of adding LMB into four plates obtained by sub-culturing human foreskin keratinocytes (HFKs) over three generations, so that the concentrations in the medium were 0 nM, 0.4 nM, 2 nM and 10 nM, respectively, in culturing of human epidermal keratinocytes. The results are shown in Fig. 1 (C).

### Example 4

Culturing of a human epidermal keratinocyte and detection of MMP-2 and MMP-9 by a gelatin zymography method were conducted by the same manner as in Example 1 except that LMB was added into four plates obtained by sub-culturing human foreskin keratinocytes (HFKs) over three generations, so that the concentrations in the medium were 0 nM, 0.4 nM, 2 nM and 10 nM, respectively, and TNF-α was added so that the four concentrations of TNF-α in the medium were all 10 ng/ml, instead of adding LMB into four plates obtained by sub-culturing human foreskin keratinocytes (HFKs) over three generations, so that the concentrations in the medium were 0 nM, 0.4 nM, 2 nM and 10 nM, respectively, in culturing of human epidermal keratinocytes. The results are shown in Fig. 1 (D).

### Example 5

Culturing of a human epidermal keratinocyte and detection of MMP-2 and MMP-9 by a gelatin zymography method were conducted by the same manner as in Example 1 except that LMB was added into four plates obtained by sub-culturing human foreskin keratinocytes (HFKs) over three generations, so that the concentrations in the medium were 0 nM, 0.4 nM, 2 nM and 10 nM, respectively, and IL-1α was added so that the four concentrations of IL-1α in the medium were all 10⁻¹⁰ M, instead of adding LMB into four plates obtained by sub-culturing human foreskin keratinocytes (HFKs) over three generations, so that the concentrations in the medium were 0 nM, 0.4 nM, 2 nM and 10 nM, respectively, in culturing of human epidermal keratinocytes. The results are shown in Fig. 1 (E).

From the results in Example 1 shown in Fig. 1 (A), it is understood that when the concentration of LMB in the medium is 2 nM or more, under no stimulation, the production of MMP-9 is inhibited. From the results of Examples 2 and 3 shown in Figs. 1 (B) and (C), it is understood that when the concentration of LMB in the medium is 2 nM or more even under conditions for stimulating differentiation by calcium at a high concentration or TGF-β and promoting the production of MMP-9, the production of MMP-9 is inhibited. Further, from the results of Examples 4 and 5 shown in Figs. 1 (D) and (E), it is understood that when the concentration of LMB in the medium is 2 nM or more even under conditions for stimulating inflammation causing by TNF-α or IL-1α and promoting the production of MMP-9, the production of MMP-9 is inhibited. From the results in Fig. 1, it is understood that LMB inhibits thepraduction of MMP-9 more specifically as compared with MMP-2.

### Example 6

The following experiment was conducted to verify that the production of mRNA of MMP-9 is inhibited, namely, the production of MMP-9 is inhibited by the action of LMB.

### <Culturing of human epidermal keratinocyte>

A foreskin of a neonate was treated at 4°C for 16 hours by a disperse having a casein decomposition activity of 25.0 caseinolytic units/ml. Epidermal was peeled from dermis using a forceps, then, the epidermal was treated with 0.05% trypsin for 5 minutes. The separated human foreskin keratinocytes (HFKs) were allowed to incubate at 37°C in a plate having a keratinocyte-SFM medium (manufactured by Invitrogen), and sub-cultured over three generations. Then, into two plates obtained by sub-culturing human foreskin keratinocytes (HFKs) over three generations, LMB was added so that the concentrations in the medium were 0 nM and 10 nM, respectively, further, allowed to incubate at 37°C for 24 hours.

### <Detection of mRNA of human MMP-9 gene and mRNA of human GAPDH gene by RT-PCR method>

Extraction of all RNAs from cultured human foreskin keratinocytes (HFKs ) was conducted according to a method of Wachi et al. (Wachi et al. (1995) FEBS Lett. 368, 215-219), and the extracted all. RNAs were stored at -80°C in the form of solution having a concentration of 1 µg/ml. A RT-PCR method was conducted using TaKaRa RNA PCR kit (manufactured by TaKaRa). For amplifying 277 base pairs of cDNA for human MMP-9, a sense primer (SEQ ID No. 1) was used for the 5' end situated at 116 to 136 base pairs, and an antisense primer (SEQ ID No. 2) was used for the 3' end situated at 372 to 392 base pairs. For amplifying 478 base pairs of cDNA for human GAPDH, a sense primer (SEQ ID No. 3) was used for the 5' end situated at 547 to 566 base pairs, and an antisense primer (SEQ ID No. 4) was used for the 3' end situated at 1005 to 1024 base pairs. Reverse transcription of RNA was conducted by incubating at 42°C for 1 hour. Incubation in PCR of cDNA obtained by reverse transcription was conducted according to the following temperature profile. Namely, for every one cycle, modification of cDNA was conducted at 94°C for 2 minutes, annealing of a primer was conducted at 60°C for 30 seconds, and synthesis of a DNA chain by a DNA polymerase was conducted at 72°C for 1 minute. A solution obtained after 30 cycles was separated, and electrophoresis was conducted using an agarose gel containing ethidium bromide, and the resulted band was observed under ultraviolet ray. The results are shown in Fig. 2. In Fig. 2, Sample 1 shows a result of use of cells having an LMB concentration in the medium of 0 nM and Sample 2 shows a result of use of cells having an LMB concentration in the medium of 10 nM.

### Example 7

Culturing of a human epidermal keratinocyte and detection of mRNA of a human MMP-9 gene and mRNA of a human GAPDH gene by a RT-PCR method were conducted by the same manner as in Example 6 except that LMB was added into two plates obtained by sub-culturing human foreskin keratinocytes (HFKs) over three generations, so that the concentrations in the medium were 0 nM and 10 nM, respectively, and a calcium chloride aqueous solution was added so that the two concentrations of Ca⁺ in the medium were all 1.5 mM, instead of adding LMB into two plates obtained by sub-culturing human foreskin keratinocytes (HFKs) over three generations, so that the concentrations in the medium were 0 nM and 10 nM, respectively, in culturing of human epidermal keratinocytes. The results are shown in Fig. 2. In Fig. 2, Sample 3 shows a result of use of cells having an LMB concentration in the medium of 0 nM and Sample 4 shows a result of use of cells having an LMB concentration in the medium of 10 nM.

### Example 8

Culturing of a human epidermal keratinocyte and detection of mRNA of a human MMP-9 gene and mRNA of a human GAPDH gene by a RT-PCR method were conducted by the same manner as in Example 6 except that LMB was added into two plates obtained by sub-culturing human foreskin keratinocytes (HFKs) over three generations, so that the concentrations in the medium were 0 nM and 10 nM, respectively, and TGF-β was added so that the two concentrations of TGF-β in the medium were all 1 ng/ml, instead of adding LMB into two plates obtained by sub-culturing human foreskin keratinocytes (HFKs) over three generations, so that the concentrations in themediumwere 0 nM and 10 nM, respectively, in culturing of human epidermal keratinocytes. The results are shown in Fig. 2. In Fig. 2, Sample 5 shows a result of use of cells having an LMB concentration in the medium of 0 nM and Sample 6 shows a result of use of cells having an LMB concentration in the medium of 10 nM.

### Example 9

Culturing of a human epidermal keratinocyte and detection of mRNA of a human MMP-9 gene and mRNA of a human GAPDH gene by a RT-PCR method were conducted by the same manner as in Example 6 except that LMB was added into two plates obtained by sub-culturing human foreskin keratinocytes (HFKs) over three generations, so that the concentrations in the medium were 0 nM and 10 nM, respectively, and TNF-α was added so that the two concentrations of TNF-α in the medium were all 10 ng/ml, instead of adding LMB into two plates obtained by sub-culturing human foreskin keratinocytes (HFKs) over three generations, so that the concentrations in the medium were 0 nM and 10 nM, respectively, in culturing of human epidermal keratinocytes. The results are shown in Fig. 2. In Fig. 2, Sample 7 shows a result of use of cells having an LMB concentration in the medium of 0 nM and Sample 8 shows a result of use of cells having an LMB concentration in the medium of 10 nM.

### Example 10

Culturing of a human epidermal keratinocyte and detection of mRNA of a human MMP-9 gene and mRNA of a human GAPDH gene by a RT-PCR method were conducted by the same manner as in Example 6 except that LMB was added into two plates obtained by sub-culturing human foreskin keratinocytes (HFKs) over three generations, so that the concentrations in the medium were 0 nM and 10 nM, respectively, and IL-1α was added so that the two concentrations of IL-1α in the medium were all 10⁻¹⁰ M, instead of adding LMB into two plates obtained by sub-culturing human foreskin keratinocytes (HFKs) over three generations, so that the concentrations in themediumwere 0 nM and 10 nM, respectively, in culturing of human epidermal keratinocytes. The results are shown in Fig. 2. In Fig. 2, Sample 9 shows a result of use of cells having an LMB concentration in the medium of 0 nM and Sample 10 shows a result of use of cells having an LMB concentration in the medium of 10 nM.

From the results in Example 6 shown in Sample 1 and Sample 2 in Fig. 2, it is understood that when the concentration of LMB in the medium is 10 nM, under no stimulation, the production of MMP-9 is inhibited, as compared with GAPDH. From the results in Example 7 shown in Sample 3 and Sample 4 in Fig. 2, it is understood that when the concentration of LMB in the medium is 10 nM, even under stimulation with calcium at a high concentration, the production of MMP-9 is inhibited, as compared with GAPDH. From the results in Example 8 shown in Sample 5 and Sample 6 in Fig. 2, it is understood that when the concentration of LMB in the medium is 10 nM, even under stimulation with TGF-β, the production of MMP-9 is inhibited, as compared with GAPDH. From the results in Example 9 shown in Sample 7 and Sample 8 in Fig. 2, it is understood that when the concentration of LMB in the medium is 10 nM, even under stimulation with TNF-α, the production of MMP-9 is inhibited, as compared with GAPDH. From the results in Example 10 shown in Sample 9 and Sample 10 in Fig. 2, it is understood that when the concentration of LMB in the medium is 10 nM, even under stimulation with IL-1α, the production of MMP-9 is inhibited, as compared with GAPDH. From the above-mentioned results, it has been clarified that addition of LMB inhibits the production of MMP-9 as compared with GAPDH.

### Example 11.

The following experiment was conducted for the purpose of checking a relation between KRE-M9 of a promoter sequence of an MMP-9 gene, the production of MMP-9, and LMB, to verify that the production of MMP-9 is inhibited by the action of LMB.

### <Culturing of human epidermal keratinocyte>

A foreskin of a neonate was treated at 4°C for 16 hours by a disperse having a casein decomposition activity of 25.0 caseinolytic units/ml. Epidermal was peeled from dermis using a forceps, then, the epidermal was treated with 0.05% trypsin for 5 minutes. The separated human foreskin keratinocytes (HFKs) were allowed to incubate at 37°C in a 24-well plate having a keratinocyte-SFM medium (manufactured by Life Technologies) containing 0.09 mM Ca⁺, and sub-cultured over three generations.

### <Preparation of vector for luciferase assay measurement>

A -73 to +16 base sequence containing KRE-M9 of an MMP-9 promoter sequence and a -56 to +16 base sequence containing no KRE-M9 were inserted into pGL3-basic vector (manufactured by Promega) which is a luciferase expression vector containing a luciferase gene according to a method of Kobayashi et al. (Kobayashi et al. (2001) EMBO Rep. 2, 604 to 608), to prepare vector-1 and vector-2. A base sequence containing a -73 to +16 base sequence containing KRE-M9 is shown in Fig. 3 (C).

### <Gene introduction>

Using the vector-1 and vector-2 and Fugene 6 (manufactured by Roche) which is a reagent for gene introduction, a vector-1 solution and a vector-2 solution each having a DNA concentration of 3 µg/1 ml were prepared, respectively. Then, the vector solution 1 was added to solutions in 12 wells and the vector solution 2 was added to solutions in the remaining 12 wells each at 37°C, of HFKs culture solutions in the 24-well plates obtained in <Culturing of human epidermal keratinocyte> and gene introduction was performed, further, 10 nM LMB was added to solutions in 6 wells to give a 6-well LMB added plate 1 and LMB was not added to solutions in the remaining 6 wells to give a 6-well LMB no-addition plate 1, of the 12 wells obtained by addition of the vector-1 solution, and 10 nM LMB was added to solutions in 6 wells to give a 6-well LMB added plate 2 and LMB was not added to solutions in the remaining 6 wells to give a 6-well LMB no-addition plate 2, of the 12 wells obtained by addition of the vector-2 solution, then, they were allowed to stand still for 3 hours. Gene introduction of a luciferase gene effected as described above induces the production of MMP-9 and the production of luciferase, and by measuring the light emitting intensity in a light emitting reaction by luciferase by a micro press counter, the production of MMP-9 can be monitored.

### <Measurement of transcription activity by luciferase assay>

Measurement of the transcription activity was conducted by a luciferase assay. The activity of luciferase was measured by Luciferase assay system (manufactured by Promega). As a micro press counter, Microbeta (manufactured by Perkin-Elmer) was used. In this measurement, the total transcription activity after 12 hours was measured using 3 wells among 6 wells, further, the total transcription activity after 24 hours was measured using the remaining 3 wells, each using the 6-well LMB added plate 1, 6-well LMB no-addition plate 1, 6-well LMB added plate 2 and 6-well LMB no-addition plate 2. The results are shown in Figs. 3 (A) and (B). In Figs. 3 (A) and (B), the upper numerical value shows a total transcription activity in the case of gene introduction of vector-1 containing KRE-M9 into HFKs, the lower numerical value shows a total transcription activity in the case of gene introduction of vector-2 containing no KRE-M9 into HFKs, and among the upper and lower numerical values, the upper stage numerical value shows a case of addition of 10 nM LMB and the lower stage numerical value shows a case of no addition of LMB, and a figure is by arbitrary unit and shows an average value of 3 wells. The standard deviation is shown by error bar.

As apparent from the results of Figs. 3 (A) and (B), when vector-1 containing KRE-M9 was gene-introduced into HFKs, the total transcription activity was reinforced with the lapse of time such as 12 hours and 24 hours after gene introduction, and by addition of LMB, Promoter activity was suppressed to about one-fifth and about one-seventh, respectively, 12 hours and 24 hours after gene introduction. When vector-2 containing no KRE-M9 was gene-introduced into HFKs, transcription activity was not detected at all. The above-mentioned facts show that KRE-M9 is important for promoting the transcription activity of MMP-9 , and that KRE-M9 regulates expression through inhibition of the transcription activity of MMP-9 in the case of the presence of LMB, supporting effectiveness of LMB as a drug for inhibiting the production of MMP-9.

### Example 12

<Culturing of human epidermal keratinocyte> was conducted in the same manner as in Example 11. <Preparation of vector for lucif erase assaymeasurement> was conducted in the samemanner as in Example 11 except that a -80 to +16 base sequence 1 containing KRE-M9 of an MMP-9 promoter sequence and TPA responsive element (hereinafter, referred to as TRE in some cases), a base sequence 2 obtained by 4-base substitution of the KRE-M9 sequence in the base sequence 1, a -714 to +16 base sequence 3, a base sequence 4 obtained by 4-base substitution of the KRE-M9 sequence in the base sequence 3 , abase sequence 5 obtained by 3-base substitution of the TRE sequence in the base sequence 4, and a base sequence 6 obtained by 4-base substitution of the KRE-M9 sequence and by 3-base substitution of the TRE sequence in the base sequence 4, were used, instead of the -73 to +16 base sequence containing KRE-M9 of an MMP-9 promoter sequence and the -56 to +16 base sequence containing no KRE-M9. The 4-base substituted sequence of the KRE-M9 sequence and the 3-base substituted sequence of the TRE sequence are shown in SEQ ID Nos. 5 and 6. <Gene introduction> and <Measurement of transcription activity by luciferase assay> were also conducted in the same manner as in Example 11 except that the total transcription activity was measured 24 hours after gene introduction. The results of measurement of transcription activity in the case of use of the base sequences 1 and 2 and the results of measurement of transcription activity in the case of use of the base sequences 3 to 6 are shown in Fig. 4 (A) and Fig. 4 (B), respectively. The upper part and lower part in Fig. 4 (A) and the first to fourth parts in Fig. 4 (B) show the total transcription activities in the case of use of the base sequence 1 and base sequence 2 and the base sequences 1 to 4, respectively, and among the upper and lower numerical values, the upper stage numerical value shows a case of addition of 10 nM LMB and the lower stage numerical value shows a case of no addition of LMB, and a figure is by arbitrary unit and shows an average value of 3 wells. The standard deviation is shown by error bar.

Further, a gel shift assay was conducted using a human keratinocyte nuclear protein according to a method of Kobayashi et al. (Kobayashi et al. (2001) EMBO Rep. 2, 604 to 608) except that a double stranded DNA1 prepared using a KRE-M9 sequence and a double stranded DNA2 prepared using a 4-base substituted sequence of a KRE-M9 sequence as depicted in SEQ ID No. 5 were used as a competitor and a KRE-M9 sequence labeled with ³²P and a 4-base substituted sequence of a KRE-M9 sequence were used as a probe in the experiment. The results are shown in Fig. 4 (C) . InFig. 4 (C), aright triangle arrow shows a band revealing detection of a probe itself and a left arrow shows a shifted band.

The results in Fig. 4 (A) show that the transcription activity of MMP-9 is suppressed by the addition of LMB in the case of use of not only the base sequence 1 but also the base sequence 2 obtained by 4-base substitution of KRE-M9, therefore, not only KRE-M9 but also TRE is important for suppression of the transcription activity of MMP-9 in a gene region containing TRE and KRE-M9. Further, the results of Fig. 4 (B) show that the transcription activity of MMP-9 is suppressed by the addition of LMB also in the case of use of the base sequence 6 varied by base substitution of both KRE-M9 and TRE, therefore, also gene regions other than KRE-M9 and TRE cause suppression of the transcription activity of MMP-9 by the addition of LMB.

From the results in Fig. 4 (C), a shifted band is detected, obtained by binding of a human keratinocyte nucleus protein to a KRE-M9 probe (first stage from left in Fig. 4 (C)). In the case of use of a double stranded DNA1 prepared by using a KRE-M9 probe and a KRE-M9 sequence as a competitor, a shifted band is not detected by competitive inhibition (second stage from left in Fig. 4 (C)). Further, in the case of use of a double stranded DNA2 prepared by using a KRE-M9 probe and a 4-base substituted sequence of a KRE-K9 sequence as a competitor, a shifted band is detected (third stage from left in Fig. 4 (C)). Additionally, in the case of use of a 4-base substituted sequence of a KRE-K9 sequence as depicted in SEQ ID No. 5 as a probe, a shifted band is not detected (second stage from right in Fig. 4 (C)) . These results teach that a human keratinocyte nucleus protein binds to a KRE-M9 sequence.

### Example 13

Ten 8-weeks old female hairless mice (Hos: HR-1) were previously bred for 1 week. Five mice (mouse A1, mouse B1, mouse C1, mouse D1, mouse E1) were irradiated at the back with ultraviolet B (UVB) of 300 mJ (about 3 MED (minimal erythema dose)), and five mice (mouse A2, mouse B2, mouse C2, mouse D2, mouse E2) were not irradiated with ultraviolet ray. Immediately after irradiation with ultraviolet ray and 24 hours after irradiation with ultraviolet ray, leptomycin B was administered by externally applying a solution of a concentration of 10 µg/ml (20 µM) to mouse B1 and mouse B2, a solution of a concentration of 1 µg/ml (2 µM) to mouse C1 and mouse C2, a solution of a concentration of 100 ng/ml (200 nM) to mouse D1 and mouse D2 and a solution of a concentration of 10 ng/ml (20 nM) to mouse E1 and mouse E2, each in an amount of 0.1 ml by a brush, as a solution containing leptomycin B dissolved in 70% ethanol. To mouse A1 and mouse A2, about 0.1 ml of 70% ethanol was applied by a brush. Then, skins were collected from mouse A1 to mouse E1 irradiated with ultraviolet ray 48 hours after irradiation and mouse A2 to mouse E2 not irradiated with ultraviolet ray. The collected skins were fixed with formalin and embedded in paraffin, then, the slices were stained with hematoxylin and eosin. Slices of skins collected from mouse A1, mouse B1, mouse C1, mouse D1 and mouse E1 and stained and slices of skins collected from mouse A2, mouse B2, mouse C2, mouse D2 and mouse E2 and stained were observed by ECLIPSE E1000M optical microscope (manufactured by NIKON), and the results are shown in Fig. 5. In Fig. 5, A1 to E1 correspond to slices of skins collected from mouse A1 to mouse E1 and stained, respectively, and A2 to E2 correspond to slices of skins collected from mouse A2 to mouse E2 and stained, respectively, and the length of a bar indicates 100 µm.

From the results in Fig. 5, it has been recognized that dyskeratosis of epidermal and inflammatory cell infiltration of dermis and subcutaneous tissue occur in the skin of mouse A1 irradiated with ultraviolet ray, while in the skins of mouse B1, mouse C1, mous.e D1 and mouse E1 irradiated with ultraviolet ray and to which leptomycin B has been externally applied, normal keratinization is manifested in epidermal in a concentration-dependent manner and inflammatory cell infiltration in dermis and subcutaneous tissue is also suppressed. Further, a significant change was not found even if leptomycin B was externally applied to the skins of mouse B2, mouse C2, mouse D2 and mouse E2 not irradiated with ultraviolet ray. Thus, suppression of inflammation of skin has been confirmed at least by external application, and effectiveness of leptomycin B on various inflammatory skin diseases and tumor has been shown. Sequence table free text

SEQ ID Nos. 1 to 4 show a sequence of a primer, and SEQ ID Nos. 5 and 6 show a 4-base substituted sequence of a KRE-M9 sequence and a substituted sequence of a TRE sequence.

### INDUSTRIAL APPLICABILITY

The drug for inhibiting the production of MMP-9 of the present invention can inhibit the production of MMP-9 more specifically as compared with MMP-2, further, can inhibit the production of MMP-9 even under stimulating the differentiation by adding calcium at a high concentration, or adding TGF-β, and under stimulating for inducing inflammation by adding TNF-α or adding IL-1α.

## Claims

1. A drug for inhibiting the production of matrix metalloproteinase-9, comprising leptomycin B of the following formula (1) 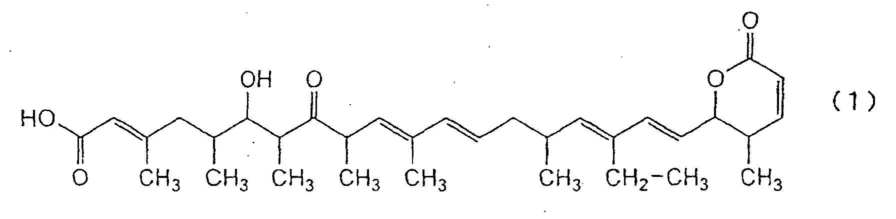 or its derivative as the active ingredient:

2. The drug according to Claim 1, which is a preventive drug or a therapeutic drug for matrix metalloproteinase-9 related skin diseases.

3. The drug according to Claim 2 wherein said preventive drug or therapeutic drug is administered in the form of skin external preparation.


SEQUENCE LISTING
SEQUENCE LISTING

<110> Japan Science and Technology Agency

<120> Medicine for inhibiting production of MMP-9

<130> YG2003-36PCT

<150> JP2002/336867
<151> 2002-11-20

<150> JP2003/136738
<151> 2003-05-15

<150> JP2003/286386
<151> 2003-08-05

<160> 6

<170> PatentIn version 3.1

<210> 1
<211> 21
<212> DNA
<213> Artificial sequence

<220>
<223> PCR Primer

<400> 1
ggagacctga gaaccaatct c 21

<210> 2
<211> 21
<212> DNA
<213> Artificial sequence

<220>
<223> PCR Primer

<400> 2
tccaataggt gatgttgtcg t 21

<210> 3
<211> 20
<212> DNA
<213> Artificial sequence

<220>
<223> PCR Primer

<400> 3
gtcatccatg acaactttgg 20

<210> 4
<211> 20
<212> DNA
<213> Artificial sequence

<220>
<223> PCR Primer

<400> 4
tgctgtagcc aaattcgttg 20

SEQUENCE LISTING

<210> 5
<211> 10
<212> DNA
<213> HFKs

<400> 5
gaattccaag 10

<210> 6
<211> 7
<212> DNA
<213> HFKs

<400> 6
tgagcat 7